Europäisches Patentamt

European Patent Office

Office européen des brevets

(11) Publication number: **0 126 580 B1**

# EUROPEAN PATENT SPECIFICATION

(45) Date of publication of patent specification: **19.11.92**   (51) Int. Cl.⁵: **A61K 9/52**

(21) Application number: **84303092.5**

(22) Date of filing: **08.05.84**

The file contains technical information submitted after the application was filed and not included in this specification

(54) **Vesicle formulations for the controlled release of therapeutic agents.**

(30) Priority: **06.05.83 US 492497**
**02.05.84 US 606450**

(43) Date of publication of application:
**28.11.84 Bulletin 84/48**

(45) Publication of the grant of the patent:
**19.11.92 Bulletin 92/47**

(84) Designated Contracting States:
**AT BE CH DE FR GB IT LI LU NL SE**

(56) References cited:
**EP-A- 0 021 337**
**EP-A- 0 072 234**

**CHEMICAL ABSTRACTS, vol. 95, no. 5, August 3, 1981, page 272, column 2, abstract 37638b**

**R. BITTMANN et al.: "Osmotic behavior of liposomes of phosphatidylcholine as a function of lipid concentration"**

(73) Proprietor: **VESTAR, INC.**
**650 Cliffside Drive**
**San Dimas California 91773(US)**

(72) Inventor: **Callahan, Richard A.**
**2007 Oak Street**
**South Pasadena California 91030(US)**
Inventor: **Tin, George Wing-Yiu**
**431 East Sandra**
**Arcadia California 91006(US)**

(74) Representative: **Goldin, Douglas Michael et al**
**J.A. KEMP & CO. 14, South Square Gray's Inn**
**London WC1R 5EU(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

**Description**

The present invention relates to formulations for controlled release in vivo of therapeutic agents. In another aspect, it relates to vesicles and, particularly, to phospholipid vesicles.

Numerous conditions in both man and lower animals are responsive to drugs or other therapeutic agents administered in vivo. To be useful, these agents must be administered at a dosage level which is high enough to cause the desired effect, and that dosage level must be maintained for a sufficient period of time to achieve that effect. Many such therapeutic agents are routinely administered by intra-muscular injection at dosage levels calculated to produce a concentration of the agent in the circulatory system which is effective. Thereafter, injections are repeated as necessary to maintain the therapeutically effective level.

It is often the case that the therapeutically effective level of the agent in circulation is maintained only a short time after injection because of breakdown of the agent by the host's defensive mechanisms against foreign substances. Moreover, the agent itself may have intolerable side effects, even lethal ones, if administered in amounts which substantially exceed the therapeutically useful level. Therefore, prolonging the effective concentration of the agent in the body by increasing the dosage is always limited by the amount of toxicity. Even in cases where toxicity is low, the size of an injection can be limited by the size of the bolus which can be administered safely.

In view of such problems, efforts have been made to develop delivery systems for therapeutic agents which may be administered in vivo and which, after administration, gradually release the agent into its environment in order to prolong the interval over which the effective concentration of the agent is maintained in that environment. In this way, the interval between administrations of the agent can be increased and, in some instances, the need for further administration can be eliminated.

One such approach to obtaining prolonged or sustained release has been to encapsulate the therapeutic agent in a "vesicle". As used herein, the term vesicle refers to a micellular particle which is usually spherical in form and which is frequently obtained from a lipid which forms a bilayered membrane and is referred to as a "liposome". Methods for making such vesicles are well known in the art. Typically, they are prepared from a phospholipid such as distearoyl phosphatidylcholine or lecithin, and may include other materials such as positively or negatively charged compounds. Vesicles made from phospholipids are commonly referred to simply as "phospholipid vesicles". Depending on the techniques for its preparation, a vesicle may form as a simple bilayered shell (a unilamellar vesicle) or it may form in multiple layers (multilamellar vesicle).

After administration, typically as a suspension in physiological saline, the vesicles gradually release the encapsulated therapeutic agent which then displays its expected effect. However, prior to its release, the agent exhibits no pharmacokinetic properties and is protected by the vesicle from metabolic degradation or other attack by the host's defense mechanisms against foreign substances. Accordingly, the agent can be safely administered in an encapsulated form in dosages which are high enough that, if directly given the host, could result in severe side effects or even death.

The time interval over which an effective concentration of the therapeutic agent is maintained after administration as a vesicle encapsulant is generally thought to be a function of the rate at which it is released from the vesicle and the rate at which it is absorbed from the point of administration after release. Since the former may vary with vesicle structure and the latter by reason of the properties of the agent, the interval over which the useful concentration of an agent in circulation is maintained can vary widely. Generally, the rate of release from the vesicle is thought to be controlling for most compounds and sustained release of encapsulated drugs over a period of 6-8 hours is a common observation. See F.J.T. Fields (1981), Liposomes: From Physical Structure to Therapeutic Applications; Research Monographs in Cell and Tissue Physiology, Vol. 7, C.G. Knight, ed., Elsevier/North Holland, N.Y., p. 51ff and R.W. Stevenson et al, Diabetologia, 19, 217 (1980). However, intramuscular injections in mice of vesicle encapsulated interferon resulted in localized levels of interferon which, after three days, were equivalent to levels observed over 2-4 hours after injection of free interferon. D.A. Eppstein et al, J. Virol., 41, 575. This longer time of sustained release likely reflects the lower mobility of this biomacromolecule from the injection site.

Notwithstanding the advance in sustained release which has been achieved using vesicles as encapsulants, further improvements in sustained release compositions are desirable to reduce still further the interval between administrations of the therapeutic agents. Even a 6-8 hour period of sustained release makes out-patient treatment difficult, if not impossible, and longer intervals would reduce the workload of hospital or other medical personnel, not to mention reducing the patient's discomfort. Furthermore, although by vesicle encapsulation the period over which an effective concentration of therapeutic agents could be maintained is extended, no effective means to control the rate of release results from encapsulation itself.

Accordingly, there remains as yet unmet, a desire for sustained release formulations of therapeutic agents which extend even further the interval over which an effective concentration of the agent is maintained.

EP-A-0 072 234 discloses that the in vitro stability of certain liposomes containing a therapeutic agent can be improved by suspending the liposomes in a continuous aqueous phase containing a pharmaceutically acceptable salt solution which is at least isotonic with human blood. This provides improved shelf life without refrigeration in tropical climates.

EP-B-0 021 337 is also concerned with in vitro stability of liposomes containing a therapeutic agent by providing at least a 0.4 molar concentration of a sugar in the liposome solution.

Bittman et al, Chemistry and Physics of Lipids, 28 (1981) 323-335 refers at page 324 to previous observations that initial rates of osmotic swelling observed on suspending isotonic or hypertonic non-electrolyte solutions are indicative of the penetration rates of the solutes through the outermost lipid bilayer.

We have now found that, by controlling the concentration of solutes in the continuous aqueous phase of an injectable liposome carrying a therapeutic agent and suspended in a continuous aqueous phase, it is possible to control the rate at which the therapeutic agent is released from the vesicle after in vivo administration by injection.

Accordingly the present invention provides a composition for the controlled sustained release in vivo of a therapeutic agent after subcutaneous or intramuscular injection to a host comprising a solution of the therapeutic agent encapsulated in vesicles, the vesicles being suspended in a solution to which has been added sufficient of a solute which is selected from sugars and polypeptides such that the suspending solution is hypertonic with respect to the solution within the vesicles and is of greater osmolarity than physiological saline.

The rate of release of the therapeutic agent from the vesicles after administration is a function of the initial osmotic pressure. Thus, as the osmolarity of the suspending solution becomes less hypotonic, relative to the solution within the vesicles, the rate of release of the therapeutic agent is slowed. Slowest releases are obtained when the suspending solution approaches an isotonic, or even hypertonic, relationship with respect to the solution within the vesicles. The compositions of the present invention exhibit a longer interval over which the sustained release of the agent is maintained compared to agents encapsulated in vesicles and administered as suspensions in physiological saline as described in the prior art.

**Descrption of the Drawings**

Figure 1. Transition Temperatures (Stability) of vesicle Formulations studied in vitro using PAC Spectroscopy.

DSPC:Chol (1:1) △—△
(2:1) □—□
(7:1) ▲—▲
DSPC:DPPC:Chol (1:1:1) X—X
(1.5: 0.5: 1) O—O
DSPC:SA:Chol(1.5:0.5:1) ■—■
DSPC:DCP:Chol(1.5:0.5:1) ●—●

Each data point is the mean of a minimum of three 15 minute incubations in the presence of rat plasma.

Figure 2: Effect of Vesicle Membrane Fluidity on Extended Release of 2-PAMCl

| A | DSPC:DPPC:Chol | (1:1:1) |
|---|----------------|---------|
| B | DSPC:DPPC:Chol | (1.5:0.5:1) |
| C | DSPC:Chol | (2:1) |

Dosage: 12 mg 2-PAMCl/rat (250 gm); 2-PAMCl: Vesicle lipid. Each data point is the average of two observations

▲—▲ plasma; ●—● whole blood.

Figure 3: Effect of vesicle lipid composition and change on extended release of 2-PAMCl

| A | DSPC:DCP:Chol | (1.5:0.5:1), negative |
|---|---------------|----------------------|
| B | DSPC:SA:Chol | (1.5 0.5:1), positive |
| C | DSPC:Chol | (2:1) neutral |

Dosage: 12 mg 2-PAMCl/rat (250 gm); 2PAMCl:vesicle lipid (12:5)

▲—▲ plasma; ●—● whole blood.

Each data point is the average of the measurements.

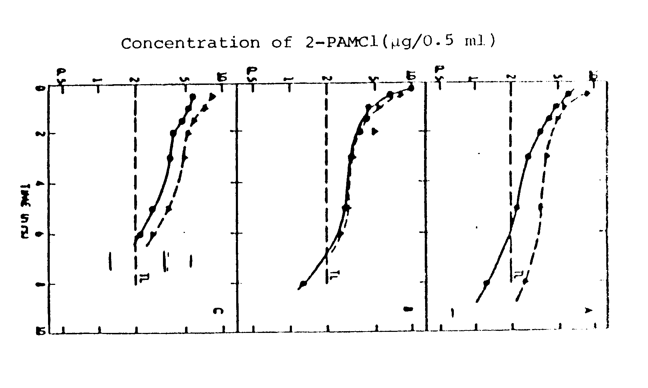

Figure 4: Effect of vesicle cholesterol content on extended release of 2-PAMCl.

| A. | DSPC:Chol | (1:1) (50 mole percent cholesterol) |
| B. | DSPC:Chol | (2:1) (33 mole percent cholesterol) |
| C. | DSPC:Chol | (7:1) (12.5 mole percent cholesterol) |

▲—▲ plasma; ●—● whole blood.

Each data point is the average of two measurements.

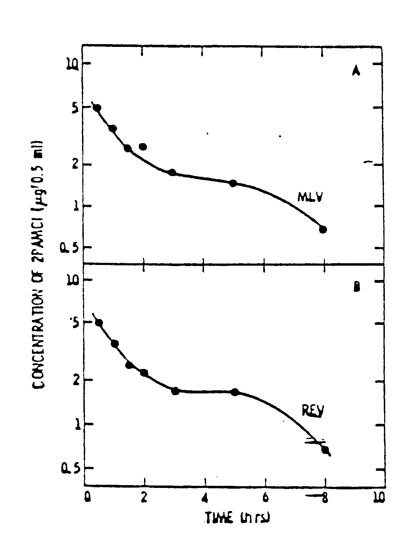

Figure 5: Extended release of 2-PAMCl by multilamellar (MLV) and large unilamellar (REV) vesicles prepared by reverse phase evaporation. Both preparations were composed of DSPC:Chol (2:1).

Dosage: 12 mg 2-PAMCl/rat (250 gm): 2-PAMCl: vesicle lipid (12:5). Values shown are whole blood concentration of 2-PAMCl.

Each data point is the mean of two measurements.

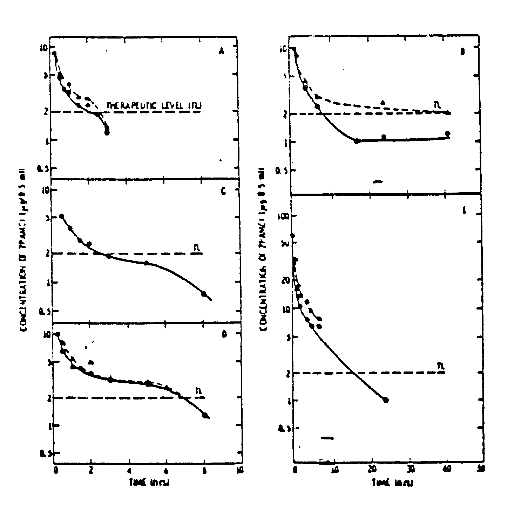

Figure 6: Effective Formulations for Extending the Therapeutic Plasma levels of 2-PAMCl injected into Muscle.

A.    12 mg 2-PAMCl in saline solution per rat.

B.    60 mg encapsulated 2-PAMCl resuspended in saline glucose solution.

C.    12 mg 2-PAMCl solution to which 2.5 mg lipid was added and formed into MLV's.

D.    12 mg 2-PAMCl solution to which 5.0 mg lipid was added and formed into MLV's.

E.    60 mg 2-PAMCl solution to which 5.0 mg lipid was added and MLV's formed using a high encapsulation technique Note high initial blood level of 2-PAMCl. Equivalent dosages of free 2-PAMCl or MLV encapsulated formulations with lower encapsulation efficiencies were fatal to all rats.

▲—▲ plasma; ●—● whole blood.

Each data point is the average of two measurements.

As noted above, the present invention provides a method for controlling the rate of release in vivo of a therapeutic agent from a vesicle encapsulant by adjusting the osmotic pressure between the solution of therapeutic agent within the vesicle and the solution in which the vesicles are suspended for parenteral administration.

Although we do not wish to be bound by any particular theory, the increase in the interval of sustained release obtained as the osmolarity of the suspending solution is made hypertonic by addition of the specific solute, relative to the solution within the vesicles, may result from the fact that, so long as the concentration of solute in the suspending solution is such that the solution is hypertonic, the internal osmotic pressure is not great enough to cause the solvent of the suspending solution to migrate into the vesicles which would increase the hydraulic pressure within the vesicles and cause them to break down or release part of their contents. After administration, however, the concentration of liquid medium around the vesicles gradually becomes more hypotonic with respect to the solution within the vesicles, for example, by absorption of the solute by the body from the suspending solution. As this occurs, the osmotic pressure between the vesicles and the hypotonic liquid medium causes liquid specifically water to migrate into the vesicles, causing release of the therapeutic agent. By contrast, the prior art practice of administering the therapeutic agent in vesicles suspended in physiological saline which is already hypotonic to the vesicles results in a much more rapid release of the vesicle contents. Accordingly, the therapeutic agent is also more rapidly released. By adjusting the osmolarity between the solution of therapeutic agent and the suspending solution, however, the rate of release can be varied giving a degree of control over this rate not hitherto attained.

In the practice of the present invention, the therapeutic agent is dissolved in a suitable solvent, for example, physiological saline, usually at or near the saturation point in the case of agents of limited solubility, and encapsulated in a suitable vesicle. Techniques to do this are well known in the art and need not be described in detail here. Presently preferred for use in the invention are multilamellar phospholipid vesicles although unilamellar vesicles and vesicles of other than phospholipid can be used, the basic essential criterion being that the material of the vesicles be tolerable by the host in the amounts to be administered.

The solution used for suspending the vesicles is preferably physiological saline (~0.15M NaCl) to which has been added the sugar or polypeptide to adjust the concentration of this solution to a hypertonic level that gives the desired rate of release. This concentration will be adjusted to that the osmoloarity of the suspending solution is hypertonic with the solution within the vesicles. Since solute is thus added to the suspending solution, it will be clear that the suspending solution will have an osmolarity greater than that of physiological saline.

The solute added is a sugar such as dextrose or hexoses such as glucose and polypeptides which do not exhibit significant biological effects. Presently preferred is glucose as it is readily obtained as a sterile substance for administration to humans and is readily absorbed by the body.

Any of a wide variety of therapeutic agents may be used as part of the invention. Among these may be mentioned antibiotics, metabolic regulators, immune modulators, toxin antidotes, etc. For example, the invention is well suited for the controlled release of antidotes to cholinesterase inhibitors.

In order to demonstrate the advantages of the present invention, there follows a description of experiments carried out with vesicle encapsulated 2-pralidoxime chloride (2-PAMCl), an agent which is a well known and thoroughly studied antidote to toxic organophosphates which inhibit cholinesterase. Persons exposed to such intoxicants in lethal amounts suffer cardiac insufficiency or respiratory paralysis which results in death. Agents such as 2-PAMCl reactivate cholinesterase if administered in a timely fashion. However, dosages of 2-PAMCl high enough to maintain the therapeutic level for a long period of time cannot be administered because undesirable side effects, even death, can result.

## EXPERIMENTAL RESULTS

## A. MATERIALS

L-$\alpha$-distearoyl phosphatidylcholine (DSPC), L-$\alpha$-dipalmitoyl phosphatidylcholine (DPPC) from Calbiochem, and cholesterol (Chol), stearylamine (SA), and dicetylphosphate (DCP) from Sigma Chemical Company were used without further purification to prepare vesicles. 2-Pyridinealdoxime (2-PAM), pralidoxime chloride (2-PAMCl) and Iodomethane were purchased from Aldrich Chemical Company and AG 1x8 ion exchange resin was from BioRad Laboratories (Richmond, CA). Ultrapure InCl$_3$ was purchased from Ventron Corporation (Danvers, MA). [$^3$H]-cholesterol oleate (specific activity: 52 Ci/mole) and [$^{14}$C]-Iodomethane (specific activity: 10 Ci/mole) were purchased from New England Nuclear. Carrier-free $^{111}$InCl$_3$ was purchased from Medi-Physics (Glendale, CA) and purified according to the method of Hwang and Mauk, Proc. Natl. Acad. Sci. USA, 74. 4991 (1977). The ionophore A23187 was from Eli Lilly and Co. Sprague-Dawley rats in the range of 200-250 g were purchased from Charles River Laboratories and kept in an AAALAC approved laboratory for one week before use in experiments.

## B. Methods

### Preparation of Vesicles

Small unilamellar vesicles (SUV's) were prepared by probe sonication of the lipid mixture in phosphate buffered saline (PBS) containing either nitrilotriacetic acid (NTA) or 2-PAMCl. See Mauk et al, Anal. Biochem., 94,302,307 (1979). A trace amount of [$^3$H] cholesterol oleate was included in the lipid mixture as a marker for the lipid phase. Following sonication, annealing, and low speed centrifugation, the NTA external to the vesicle was removed by passage of the preparation over a Sephadex G-50 column, equilibrated with PBS.

Large unilamellar vesicles (LUV's) were prepared by the reverse phase evaporation (REV) method described by Szoka and Papahadjopoulos, Proc. Natl. Acad. Sci. USA, 75, 4194 (1978). REV vesicles are formed when an aqueous buffer containing the material to be encapsulated is introduced into a mixture of phospholipid and organic solvent, and the organic solvent is subsequently removed by evaporation under reduced pressure. The REV vesicles are then passed through a gel permeation column to remove the solvent residue and the unencapsulated drug.

Multilamellar vesicles (MLV's) were prepared by stirring the dry lipid film with the material to be encapsulated. Free unencapsulated materials can be separated from MLV encapsulated material by centrifugation at 12,000 xg. In our preparation of MLV's for in vivo injection, 40 mg of DSPC:Chol (2:1 molar ratio) (or other compositions as indicated) were stirred for 1/2 hour in a round bottom flask with 1-2 ml of PBA containing 0.5 M 2-PAMCl, or 3M 2-PAMCl as indicated.

### Synthesis of [$^{14}$C] 2-PAMCl

Radiolabeled 2-PAMCl for use in the studies could not be obtained from any source. Therefore, the radiolabeled drug was synthesized. [$^{14}$C] labeled 2-Pralidoxime Iodide (2-PAMI) was first synthesized by refluxing 2-pyridine aldoxime (2-PAM) with [$^{14}$C]-methyl iodide in nitrobenzene for three hours at 75-80°C. The reaction was then stopped, and the yellow precipitate of 2-PAMI was filtered and recrystallized from methanol. These yellow crystals of 2-PAMI were then dissolved in a minimal amount of water and passed through an anionic exchange column (BioRad AG 1x8). The chloride salt of 2-PAM was isolated by drying the solution with a rotary evaporator followed by recrystallization from ethanol. Approximately 1.5 gm of pure [$^{14}$C] labeled 2-PAMCl was obtained with specific activity of 25 $\mu$ Ci/ mole. The chemical identity of this material was confirmed by (1) the melting point of the compound which was found to be 232-234°C (literature value 235°C) and (2) the characteristic absorption of an acidic solution of 2-PAMCl at approximately 295, 245, and 210 nm; and (3) co-migration during thin layer chromotcgraphy of the $^{14}$C labeled compound with 99% pure 2-PAMCl.

### In Vitro Vesicle Studies

The chemical structure of vesicles was altered by varying the length of their phosphatidylcholine carbon chain, cholesterol content, and surface charge. The stability of the vesicle formulations in vitro was studied by gamma-ray perturbed angular correlation spectroscopy (PAC) as described by Hwang and Mauk, Proc. Natl. Acad. Sci. USA, 74, 4991 (1977). Prior to PAC measurements the vesicles were loaded with $^{111}\overline{\text{InCL}_3}$ Typically 1.0 mg of vesicles with the ionophore A23187 incorporated within the bilayer was incubated with $^{111}$InCL$_3$ at 80°C for 45 minutes. During incubation, the $^{111}$In passed through the ionophore and complexed with NTA inside the vesicles. The remaining $^{111}$In outside the vesicle was subsequently complexed to EDTA and separated from the loaded vesicles by chromatographing the mixture on a Sephadex G-50 column equilibrated with PBS. The vesicles, now loaded with $^{111}$In-NTA, were then suspended in a 1:1 solution of physiological saline and rat plasma. Gamma-ray PAC spectroscopy was then used to monitor the structural integrity of vesicles by measuring the tumbling rate of $^{111}$In$^{+3}$.

$^{111}$In$^{+3}$ chelted to nitrilotriacetic acid exhibits a fast tumbling rate. However, upon disruption of the vesicle, the released $^{111}$In$^{+3}$ rapidly binds to macromolecules in the surrounding medium and exhibits a markedly decreased tumbling rate. Repeated PAC measurements of each vesicle formulation were used to estimate vesicle stability based on the time course for the release of $^{111}$In.

Similarly, identical vesicle formulations loaded with [$^{14}$C]-2-PAMCl were used to measure the release rate of 2-PAMCl. Aliquots were periodically withdrawn from each preparation and free 2-PAMCl separated from the microencapsulated drug by gel filtration chromatography as described by Huang, Biochem, 8, 344 (1969). The amount of [$^{14}$C]-2-PAMCl remaining within the vesicles was measured by liquid scintillation counting.

### In Vivo Vesicle Studies

The rate of release of [$^{14}$C]-2-PAMCl from the various vesicle formulations was measured in vivo using male Sprague-Dawley rats obtained from Charles River Inc. Dosages ranging from 5-240 mg/kg body weight (BW) of free and encapsulated [$^{14}$C]-2-PAMCl were injected into the thigh muscle. Individual injection volumes never exceeded 0.15 ml. At scheduled times after injection, the rats were either sacrificed or bled through the eye orbit. Radiolabeled 2-PAMCl was measured in blood and plasma by liquid scintillation counting using the New England Nuclear procedure for counting labeled biological material. See L.S.C. Note, #44, New England Nuclear Applications Laboratory, Boston, MA.

### C. Results

### In Vitro Vesicle Stability

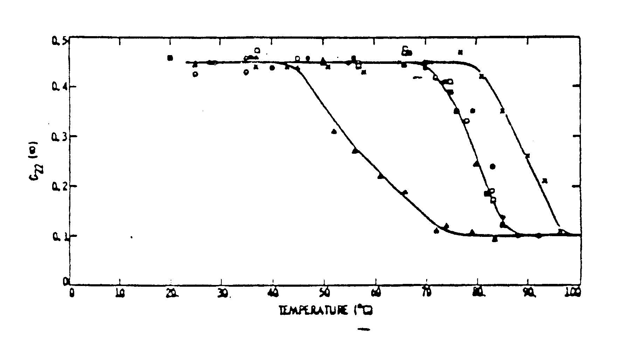

As indicated in the previous section, the rate of $^{111}$In release from vesicles in the presence of plasma can be monitored by PAC spectroscopy. Figure 1 shows the percent $^{111}$In remaining encapsulated with seven vesicle formulations varying in cholesterol concentration, carbon chain length, and surface charge. With the exception of the negatively-charged DCP vesicle, all vesicle formulations with 33 mole percent or more cholesterol exhibited the same transition temperature as monitored by $^{111}$In release. The DCP formulation produced vesicles with a transition temperature ($^{111}$In release) approximately 10°C higher than other vesicle formulations containing the same amount of cholesterol.

### In Vivo Results

In the following results, the data are presented as the concentration of 2-PAMCl as a function of time for various vesicle formulations. The time dependence for a standard injection of free 2-PAM Cl shows that the blood concentration drops below therapeutic level (TL) in 2-3 hrs (Fig 6A). As will be shown, all vesicle formulations with encapsulated 2-PAMCl exhibited extended blood levels. Therapeutic levels of drug were maintained typically 6-8 hrs for those formulations having only buffered saline as the suspending medium. Vesicles suspended in a high osmolar medium showed dramatically longer therapeutic levels.

The Effect of Altering the Chemical Composition of the Vesicle Membrane

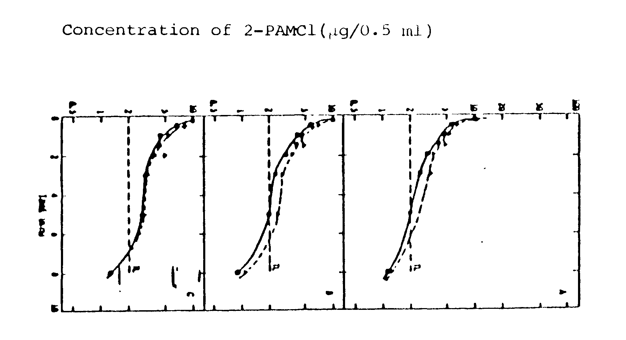

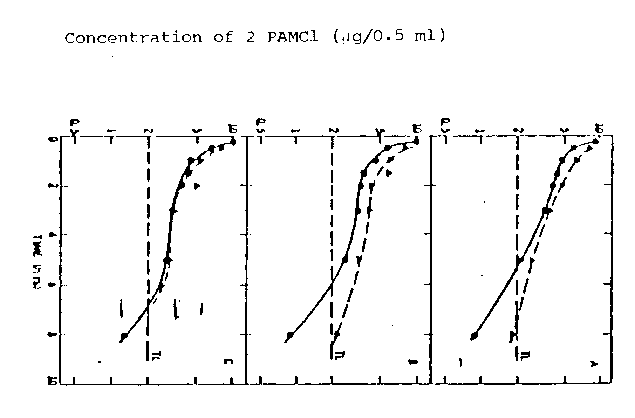

Altering membrane fluidity by changing lipid composition did not effect the extended release period achieved with all vesicle formulations (Fig. 2). Similarly, altering the lipid composition and charge of the vesicle membrane did not significantly alter the release period either. (Fig. 3).

Among the vesicle composition and charge variables studied, the only factor affecting the release rate for 2-PAMCl was cholesterol content. Increasing the cholesterol content of vesicle membranes from 12.5 - 50.0 mole percent appeared to slightly lengthen the time therapeutic levels of 2-PAMCl remained in circulation (Fig. 4).

The Effects of Altering the Physical Structure of Vesicles

The effect of vesicle structure on the extended release of 2-PAMCl was examined using multilamellar vesicles (MLV's) and large unilamellar vesicles (LUV-s) prepared by reverse phase evaporation (REV) vesicles. As shown in Fig. 5, DSPC: Cholesterol vesicles possessing the two distinct structures exhibited essentially identical 2-PAMCl release properties. Consequently MLV's containing 30 mole percent cholesterol were used in the high osmolarity studies as described below.

The Effects of Altering Encapsulation Volume and Using High Osmolar Suspending Solution

The above data show that there are comparatively small changes in the time dependency of 2-PAMCl blood-levels for the formulations tested. The results indicate that simple manipulation of vesicle composition and morphology are not likely to provide extended release beyond 6-8 hrs. These results are consistent with other published observations which show modest extended release times.

Presented below are results which show that blood levels of 2-PAMCl can be dramatically extended by increasing the osmolarity of the medium in which the vesicles are suspended. Also, proportionately higher concentrations of drug can be encapsulated without leakage from the vesicles.

The most effective vesicle formulations for extending the therapeutic plasma levels (4.0 $\mu$g/ml plasma) in rats was found to be a 2:1 DSPC:Cholesterol lipid mixture, formed as MLV's and encapsulating a 3 molar solution of [$^{14}$C]-2-PAMCl which was suspended in an isomolar glucose-physiological saline solution 3M in glucose, after working to remove mother liquor. In this case, the osmolarity of the suspending medium is about 60% of the osmolarity of the encapsulated drug. Intramuscular injections (0.15ml) of this vesicle formulation extended the therapeutic plasma drug level from 2-1/4 hours, obtained with the conventional saline formulation, (Fig. 6A), to at least 40 hours (Fig. 6B). Animals receiving the isomolar vesicle formulation exhibited no toxic symptoms and their blood drug levels never exceeded the 20 $\mu$g/ml level achieved by control animals receiving the standard 12 mg 2-PAMCl saline formulation (Fig. 6A).

The extension of therapeutic blood levels is related to the amount of drug encapsulated. All encapsulation techniques extended the maintenance of therapeutic drug levels. Doubling the encapsulating lipid material from 2.5 mg to 5.0 mg by doubling the quantity of lipid (and thereby increasing encapsulated volume of drug solution) increased the time that therapeutic levels were maintained from 2.5 to 7.0 hours (Fig. 6, C,D). Similarly increasing the amount of drug encapsulated from a saline solution containing 60 mg of 2-PAMCl prevented all acute toxic symptoms while extending the therapeutic blood titers to 15 hours (Fig. 6 E). All animals receiving similar 60 mg injections of 2-PAMCl unencapsulated in saline solution, or encapsulated using a technique which reduces the encapsulation efficiency died within 30 minutes after injection.

These data suggest that encapsulated drug acts as a third compartment from which its slow release lowers the peak blood levels seen when equal, unencapsulated dosages are injected.

**Claims**
**Claims for the following Contracting States : BE, CH, DE, FR, GB, IT, LI, LU, NL, SE**

EP 0 126 580 B1

1. A composition for the controlled sustained release in vivo of a therapeutic agent after subcutaneous or intramuscular injection to a host comprising a solution of the therapeutic agent encapsulated in vesicles, the vesicles being suspended in a solution to which has been added sufficient of a solute which is selected from sugars and polypeptides such that the suspending solution is hypertonic with respect to the solution within the vesicles and is of greater osmolarity than physiological saline.

2. A composition according to Claim 1 wherein the solute is a sugar.

3. A composition according to Claim 2 wherein the sugar is glucose or another hexose.

4. A process for producing a composition as defined in any one of the preceding claims, comprising encapsulating a solution of the therapeutic agent in vesicles and suspending the vesicles in a solution to which has been added sufficient of a solute which is selected from sugars and polypeptides such that the suspending solution is hypertonic with respect to the solution within the vesicles and is of greater osmolarity than physiological saline.

5. A compostion as defined in any one of Claims 1 to 3 for use in a method for the treatment of the human or animal body by surgery or therapy or in a diagnostic method practised on the human or animal body.

**Claims for the following Contracting State : AT**

1. A process for producing a composition for the controlled sustained release in vivo of a therapeutic agent after subcutaneous or intramuscular injection to a host, comprising encapsulating a solution of the therapeutic agent in vesicles and suspending the vesicles in a solution to which has been added sufficient of a solute which is selected from sugars or polypeptides such that the suspending solution is hypertonic with respect to the solution within the vesicles and is of greater osmolarity than physiological saline.

2. A process according to Claim 1 wherein the solute is a sugar.

3. A process according to Claim 2 wherein the sugar is glucose or another hexose.

4. A composition prepared as in any one of Claims 1 to 3 for use in a method for treatment of the human or animal body by surgery or therapy or in a diagnostic method practised on the human or animal body.

**Patentansprüche**
**Patentansprüche für folgende Vertragsstaaten : BE, CH, DE, FR, GB, IT, LI, LU, NL, SE**

1. Zusammensetzung zur gesteuerten, andauernden in vivo-Abgabe eines therapeutischen Mittels nach subkutaner oder intramuskulärer Injektion in einen Wirt enthaltend eine Lösung eines in Bläschen verkapselten therapeutischen Mittels, wobei die Bläschen in einer Lösung suspendiert sind, zu der eine ausreichende Menge eines gelösten Stoffes zugesetzt worden ist, dar ausgewählt ist aus zuckern und Polypeptiden, so daß die suspendierende Lösung hypertonisch bezüglich der Lösung innerhalb der Bläschen ist und eine größere Osmolarität als physiologische Kochsalzlösung aufweist.

2. Zusammensetzung nach Anspruch 1, wobei der gelöste Stoff ein Zucker ist.

3. Zusammensetzung nach Anspruch 2, wobei der Zucker Glukose oder eine andere Hexose ist.

4. Verfahren zum Herstellen einer Zusammensetzung gemäß Definition in einem der vorhergehenden Ansprüche, bei dem man eine Lösung des therapeutischen Mittels in Bläschen verkapselt und die Bläschen in einer Lösung suspendiert, zu der eine ausreichende Menge eines gelösten Stoffes zugesetzt worden ist, der ausgewählt ist aus Zuckern und Polypeptiden, so daß die suspendierende Lösung hypertonisch bezüglich der Lösung innerhalb dar Bläschen ist und eine größere Osmolarität als physiologische Kochsalzlösung aufweist.

8

**5.** Zusammensetzung nach einem der Ansprüche 1 bis 3 zur Anwendung in einem Verfahren zur Behandlung des menschlichen oder tierischen Körpers durch Chirugie oder Therapie oder in einem am menschlichen oder tierischen Körper angewandten diagnostischen Verfahren.

**Patentansprüche für folgenden Vertragsstaat : AT**

**1.** Verfahren zum Herstellen einer Zusammensetzung zur gesteuerten, andauernden in vivo-Abgabe eines therapeutischen Mittels nach subkutaner oder intramuskulärer Injektion in einen Wirt, bei dem man eine Lösung eines therapeutischen Mittels in Bläschen verkapselt und die Bläschen in einer Lösung suspendiert, zu der eine ausreichende Menge eines gelösten Stoffes zugesetzt worden ist, der ausgewählt ist aus Zuckern und Polypeptiden, so daß die suspendierende Lösung hypertonisch bezüglich der Lösung innerhalb der Bläschen ist und eine größere Osmolarität als physiologische Kochsalzlösung aufweist.

**2.** Zusammensetzung nach Anspruch 1, wobei der gelöste Stoff ein Zucker ist.

**3.** Zusammensetzung nach Anspruch 2, wobei der Zucker Glukose oder eine andere Hexose ist.

**4.** Zusammensetzung, hergestellt gemäß einem der Ansprüche 1 bis 3 zur Anwendung in einem Verfahren zur Behandlung des menschlichen oder tierischen Körpers durch Chirugie oder Therapie oder in einem am menschlichen oder tierischen Körper angewandten diagnostischen Verfahren.

**Revendications**
**Revendications pour les Etats contractants suivants : BE, CH, DE, FR, GB, IT, LI, LU, NL, SE**

**1.** Composition pour la libération contrôlée in-vivo d'un agent thérapeutique après injection sous cutanée ou intramusculaire à un sujet comprenant une solution de l'agent thérapeutique encapsulé dans des vésicules, les vésicules étant en suspension dans une solution à laquelle a été ajoutée une quantité suffisante d'un soluté choisi parmi les sucres et les polypeptides de telle sorte que la suspension soit hypertonique par rapport à la solution à l'intérieur des vésicules et soit d'osmolarité plus grande qu'une solution saline physiologique.

**2.** Composition selon la revendication 1 dans laquelle le soluté est un sucre.

**3.** Composition selon la revendication 2 dans laquelle le sucre est du glucose ou un autre hexoce.

**4.** Procédé pour la préparation d'une composition telle que définie selon l'une quelconque des revendications précédentes comprenant l'encapsulation d'une solution de l'agent thérapeutique dans des vésicules et la mise en suspension des vésicules dans une solution à laquelle a été ajoutée une quantité suffisante d'un soluté choisi parmi les sucres et les polypeptides de telle sorte que la suspension soit hypertonique par rapport à la solution a l'intérieur des vésicules et soit d'osmolarité plus grande qu'une solution saline physiologique.

**5.** Composition selon l'une quelconque des revendications 1 à 3 destinée à être utilisée dans un procédé pour le traitement du corps humain ou animal par chirurgie ou thérapie ou dans une méthode de diagnostic pratiquée sur le corps humain ou animal.

**Revendications pour l'Etat contractant suivant : AT**

**1.** Procédé pour la préparation d'une composition pour la libération contrôlée in-vivo d'un agent thérapeutique après injection sous cutanée ou intramusculaire à un sujet comprenant l'encapsulation d'une solution d'un agent thérapeutique dans des vésicules, et la mise en suspension des vésicules dans une solution dans laquelle a été ajoutée une quantité suffisante d'un soluté choisi parmi les sucres ou les polypeptides de telle sorte que la suspension soit hypertonique par rapport à la solution à l'intérieur des vésicules et soit d'osmolarité plus grande qu'une solution saline physiologique.

**2.** Procédé selon le revendication 1 dans laquelle le soluté est un sucre.

3. Procédé selon la revendication 2 dans laquelle le sucre est du glucose ou un autre hexoce.

4. Composition obtenue selon l'une quelconque des revendications 1 à 3 destinée à être utilisée dans une méthode pour le traitement du corps humain ou animal par chirurgie ou thérapie ou dans une méthode de diagnostic pratiquée sur le corps humain ou animal.